# EUROPEAN PATENT APPLICATION

(11) **EP 1 600 456 A1**
(43) Date of publication of application: **30.11.2005**
(21) Application number: 04012720.1
(22) Date of filing: 28.05.2004
(51) Int. Cl.: C07K 1/20, C07K 1/22

(54) **Method for the purification of polymers carrying a lipophilic group**

(71) Applicant: Deutsches Krebsforschungszentrum, 69120 Heidelberg (DE)
(72) Inventor: Jacob, Annette, 69221 Dossenheim (DE); Hoheisel, Jörg, 69168 Wiesloch (DE); Brandt, Ole, 69126 Heidelberg (DE)
(74) Representative: Vossius & Partner

(57) **Abstract**

This invention relates to a method of producing a purified polymer selected from the group consisting of PNAs, (poly)peptides, PNA chimera, peptide-DNA chimera, and derivatives thereof, wherein the polymer carries at least one lipophilic group and wherein the method comprises the following steps: (a) transferring a solution comprising the polymer carrying a lipophilic group to a lipophilic surface under conditions that allow binding of said lipophilic group to said lipophilic surface; (b) washing said surface under conditions that allow said binding to be maintained, wherein polymers not carrying said lipophilic group are removed; (c) washing said surface under conditions that break said binding; (d) collecting the washing solution from step (c); and (e) obtaining said purified polymer from said washing solution. Preferably, the lipophilic group is a protection group, or a label such as a fluorescent dye, or a linker, for example a linker suitable for immobilization on a support.

## Description

This invention relates to a method of producing a purified polymer selected from the group consisting of PNAs, (poly)peptides, PNA chimera, peptide-DNA chimera, and derivatives thereof, wherein the polymer carries at least one lipophilic group and wherein the method comprises the following steps: (a) transferring a solution comprising the polymer carrying a lipophilic group to a lipophilic surface under conditions that allow binding of said lipophilic group to said lipophilic surface; (b) washing said surface under conditions that allow said binding to be maintained, wherein polymers not carrying said lipophilic group are removed; (c) washing said surface under conditions that break said binding; (d) collecting the washing solution from step (c); and (e) obtaining said purified polymer from said washing solution. Preferably, the lipophilic group is a protection group, or a label such as a fluorescent dye, or a linker, for example a linker suitable for immobilization on a support.

In this specification, a number of documents is cited. The disclosure of these documents, including manufacturer's manuals, is herewith incorporated by reference in its entirety.

Polymers can be classified into polymers made from one type of monomer and polymers made from more than one type of monomer. The latter class may contain the monomers in stochastic order, or may exhibit a defined sequence of monomers. Polymers made from more than one monomer and exhibiting a defined sequence can be synthesized in a stepwise manner, such that the polymer chain grows by one monomer in each step. This involves adding of the required monomer in each respective step under conditions that reaction, i.e., formation of a covalent bond with the growing end of the polymer chain, can occur. Depending on the chemical nature of the monomers and the growing polymer, it may occur that not only those groups of the monomer to be added and of the growing polymer that are involved in forming the desired covalent bond, but also other groups are capable to react under said conditions. In order to ensure that only the desired bond is formed in such cases, methods using protection groups have been developed. The purpose of a protection group is to convert a reactive group into a group which is not any more reactive under defined conditions. A further prerequisite is that the protection group can be removed under conditions leaving the newly formed bond(s) connecting the monomers intact. Synthesis of (poly)peptides using protection groups is reviewed, for example, in Jarowicki and Kocienski (2000) and Kocienski (1994), that of PNAs for example in Casale et al. (1999) and Koch (1999).
Alternatively to synthesis from monomers only, the synthesis may also involve use of larger building blocks such as dimers, trimers, tetramers or even larger fragments. Synthesis may occur only using these larger building blocks or may involve monomers and larger building blocks.

Despite its great success, the above described method of synthesizing polymers has also its drawbacks. As each of the involved reactions, i.e., bond formation, acetylation, de-protection etc. are generally characterized by a yield below 100%, unwanted by-products are formed during synthesis. By-products comprise polymeric species lacking one or more monomers. As a consequence, when the pure polymer is to be obtained, one or more purification steps have to be performed. Depending on the length of the polymer and the physico-chemical characteristics of the polymer and the by-products of shorter length, the task of purification may be more or less intricate. For example, one or more by-products of only slightly shorter length may exhibit physico-chemical properties very similar to those of the desired full-length product.
However, there is another reason why the purification step is cumbersome. While the synthesis involving protection groups can be performed in parallel and with high throughput, for example on a solid support such as a resin, the purification methods described in the art are less amenable to high throughput. Typically, and as well-known in the art, standard HPLC is used for purifying the polymer. This approach is laborious, time-consuming and costly and is not suited for high throughput.
Notwithstanding the general considerations made above, distinct properties of different polymers imply different strategies of synthesis and purification.

Oligonucleotides are obtainable from solid phase synthesis using CPG (controlled pore glass) as a solid support. They have a negatively charged sugar-phosphate backbone. Accordingly, one routine method of purification applied is ion exchange HPLC of the completely de-protected ("trityl-off") oligonucleotides. Ion exchange HPLC is characterized by very high separation power, however, is laborious because it necessitates a further step, viz. de-salting of the sample after purification.
Alternatively, the 5'-OH group remains protected by the dimethoxytrityl group used as protection group during synthesis ("trityl-on"), and reversed phase (RP) HPLC is used to purify the desired product. By leaving the trityl group, i.e. a highly lipophilic group, attached to the oligonucleotide, i.e. a hydrophilic molecule, the overall properties of the molecule differ significantly from the de-protected product and the by-products not carrying the trityl group. The use of RP-HPLC does not entail the need for de-salting after purification and provides a separation power which is nearly as good as that of ion exchange HPLC. Therefore, it is the method of purifying oligonucleotides which is most applied most commonly. Typically, it is performed in column format (see for example Becker et al. (1985); columns can be obtained, for example, from Vydac®), which limits throughput. More recently, gravity flow columns and small columns which can be arranged in microtiter plate format, both with reversed phase material for trityl-on oligonucleotide purification, have become available.

Peptides and PNAs on the other hand have an electrostatically neutral backbone. They are obtainable by solid-phase synthesis on a resin. In the last synthesis step, the peptides are de-protected and cleaved off from the resin. The completely de-protected raw products are purified using RP-HPLC. As an eluent, typically an acetonitrile (ACN) gradient in a 0.1 % aqueous solution of trifluoroacetic acid (TFA) is used. The bottleneck created by a purification step effected by HPLC has been recognized and first steps towards parallel and high-throughput purification of polymers have been undertaken. Such approaches are, for example, reversed-phase sample displacement chromatography, wherein up to 24 sample can be handled in parallel (Husband et al. (2000)), and ion pair reversed-phase solid-phase extraction (IP-RP-SPE) in 96-well plates (Pipkorn et al. (2002)). The latter approach, however, is not applicable, for example, for all conceivable peptides in view of their greatly varying properties. Furthermore, purification is not always satisfactory, in particular in case of similar physico-chemical characteristics of the peptide or PNA and the by-products of shorter length. In such a case, the discriminatory power may be insufficient.

In view of the limitations of the methods described in the prior art, the technical problem underlying the present invention was to provide means and methods for the purification of polymers selected from the group consisting of PNAs, (poly)peptides, PNA chimera, peptide-DNA chimera, and derivatives thereof, wherein said purification exhibits satisfactory discriminatory power and can be effected in parallel and with high throughput.

Accordingly, this invention relates to a method of producing a purified polymer selected from the group consisting of PNAs, (poly)peptides, PNA chimera, peptide-DNA chimera, and derivatives thereof, wherein the polymer carries at least one lipophilic group and wherein the method comprises the following steps: (a) transferring a solution comprising the polymer carrying a lipophilic group to a lipophilic surface under conditions that allow binding of said lipophilic group to said lipophilic surface; (b) washing said surface under conditions that allow said binding to be maintained, wherein polymers not carrying said lipophilic group are removed; (c) washing said surface under conditions that break said binding; (d) collecting the washing solution from step (c); and (e) obtaining said purified polymer from said washing solution.

The term "polymer" as used herein relates to a compound formed from monomers, wherein, when a bond is formed between two monomers or a monomer and the growing polymer, either only a polymer extended by one unit is formed or a low molecular weight product, for example H₂O, is formed in addition. The latter group of polymers is also referred to as polycondensates. The term "polymer" as used herein deliberately comprises polycondensates. *Mutatis mutandis* the above said applies also to synthesis involving larger building blocks such as dimers or trimers in addition to or instead of monomers.

The term "(poly)peptide" as used herein describes proteins or fragments thereof and refers to a group of molecules which comprise the group of oligopeptides, consisting of two to nine amino acids, as well as the group of polypeptides, consisting of 10 or more amino acids. Preferably, the length of the polypeptide does not exceed about 100 amino acids.

PNA stands for "peptide nucleic acid". In brief, a PNA is nucleic acid, wherein the sugar-phosphate backbone has been replaced with an amide backbone. Preferably, the length of PNAs according to the invention ranges from dimers to 100-mers, more preferred from dimers to 50-mers.
PNA oligomers are synthetic DNA-mimics with an amide backbone (Nielsen et al. (1991), Egholm et al. (1993)) that exhibit several advantageous features. They are stable under acidic conditions and resistant to nucleases as well as proteases (Demidov et al. (1994)). Their electrostatically neutral backbone increases the binding strength to complementary DNA compared to the stability of the corresponding DNA duplex (Wittung et al. (1994), Ray and Norden (2000)). Thus, PNA oligomers can be shorter than oligonucleotides when used as hybridisation probes. On the other hand, mismatches have a more destabilising effect, thus improving discrimination between perfect matches and mismatches. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to the probe molecules.

PNA chimera according to the present invention are molecules comprising one or more PNA portions. The remainder of the chimeric molecule may comprise one or more DNA portions (PNA-DNA chimera) or one or more (poly)peptide portions (peptide-PNA chimera). Peptide-DNA chimera according to the invention are molecules comprising one or more (poly)peptide portions and one or more DNA portions. Molecules comprising PNA, peptide and DNA portions are envisaged as well. The length of a portion of a chimeric molecule may range from 1 to n-1 bases, equivalents thereof or amino acids, wherein "n" is the total number of bases, equivalents thereof and amino acids of the entire molecule.

The term "derivatives" relates to the above described PNAs, (poly)peptides, PNA chimera and peptide-DNA chimera, wherein these molecules comprise one or more further groups or substituents different from PNA, (poly)peptides and DNA. All groups or substituents known in the art and used for the synthesis of these molecules, such as protection groups, and/or for applications involving these molecules, such as labels and (cleavable) linkers are envisaged.

All fields of research in need of PNAs or purified (poly)peptides benefit from the method of the invention. Examples for the use of (poly)peptides are peptide libraries which are used in screens for lead compounds, for example for therapeutic applications; analysis of protein function including molecular interactions and analysis of enzyme function and specificity, for example phosphorylation studies. Specific applications of PNAs include FISH (Rigby et al. (2002), Larsen et al. (2003)), molecular beacons (Kuhn et al. (2002)), PCR-clamping (Orum et al. (1993). Murdock et al. (2000), Sun et al. (2002)), PNA-openers (Demidov and Frank-Kamenistskii (2002)), PNAs with specific sequences coupled to beads for purification of DNA, and PNA-arrays.

The term "purified" denotes at least 85% pure, preferably at least 90% or 95% pure, more preferably at least 98%, 99% or 99.5% pure, and most preferred at least 99.9% pure polymer. Purification comprises the partial or complete removal of by-products of the synthesis. Said by-products comprise polymers of shorter length than the desired polymer, i.e., polymers lacking one or more monomers.

The term "lipophilic" relates to a property of the group attached to the polymer. It denotes a preference for lipids (literal meaning) or for organic or apolar liquids or for liquids with a small dipole moment as compared to water. The term "hydrophobic" is used with equivalent meaning herein.
The mass flux of a molecule at the interface of two immiscible or substantially immiscible solvents is governed by its lipophilicity. The more lipophilic a molecule is, the more soluble it is in the lipophilic organic phase. The partition coefficient of a molecule that is observed between water and n-octanol has been adopted as the standard measure of lipophilicity. The partition coefficient P of a species A is defined as the ratio P = [A]_{n-octanol} / [A]_{water}. A figure commonly reported is the logP value, which is the logarithm of the partition coefficient. In case a molecule is ionizable, a plurality of distinct microspecies (ionized and not ionized forms of the molecule) will in principle be present in both phases. The quantity describing the overall lipophilicity of an ionizable species is the distribution coefficient D, defined as the ratio D = [sum of the concentrations of all microspecies]_{n-octanol} / [sum of the concentrations of all microspecies] water. Analogous to logP, frequently the logarithm of the distribution coefficient, logD, is reported.
If the lipophilic character of a substituent on a first molecule is to be assessed and/or to be determined quantitatively, one may assess a second molecule corresponding to that substituent, wherein said second molecule is obtained, for example, by breaking the bond connecting said substituent to the remainder of the first molecule and connecting (the) free valence(s) obtained thereby to hydrogen(s).
Alternatively, the contribution of the substituent to the logP of a molecule may be determined. The contribution πₓ of a substituent X to the logP of a molecule R-X is defined as πₓ = logP_{R-X}-logP_{R-H}, wherein R-H is the unsubstituted parent compound. Values of P and D greater than one as well as logP, logD and πₓ values greater than zero indicate lipophilic/hydrophobic character, whereas values of P and D smaller than one as well as logP, logD and πₓ values smaller than zero indicate hydrophilic character of the respective molecules or substituents.
The above described parameters characterizing the lipophilicity of the lipophilic group according to the invention can be determined by experimental means and/or predicted by computational methods known in the art (see for example Sangster (1997)).

The term "binding" as used herein refers to non-covalent interactions. It comprises adsorption processes.

Conditions suitable for steps (a), (b) and (c), respectively are well known in the art. For example, increasing concentrations of acetonitrile, methanol, ethanol or dimethylformamide (DMF) can be used for establishing appropriate conditions. Exemplary concentrations are shown in the Examples enclosed herewith. Alternative to a jump in concentration of, for example, acetonitrile, gradients of the eluens may be applied.

In a preferred embodiment, said lipophilic group is a protection group.

In a more preferred embodiment, said protection group is a protection group used in the synthesis of the polymer. Accordingly, the polymer is obtained in protected form from the synthesis.

In a preferred embodiment, said lipophilic group is 9-Fluorenyl-methyl-oxy-carbonyl (9-Fluorenylmethoxy-carbonyl, Fmoc). This specific embodiment is referred to as Fmoc-on purification.

It is understood that the methods of synthesizing polymers in a stepwise manner from building blocks such as monomers yield a full-length polymer carrying a terminal protection group and by-products of shorter length, which do not carry this protection group (but may still carry other protection groups).

A protection group according to the present invention is a group which, when bonded to a reactive group, converts said reactive group into a group which is not any more reactive under defined conditions. Furthermore, the protection group can be removed under conditions leaving the newly formed bond(s) connecting the building blocks intact. Protection groups and their use in chemical synthesis are well known in the art and described, for example, in Jarowicki and Kocienski (2000), Kocienski (1994), Casale et al. (1999) and Koch (1999).

The prior art methods for purifying peptides or PNAs subsequent to their synthesis involve the step of de-protecting the polymer prior to purification. At first sight, this measure appears logical, noting that after completion of synthesis and during purification generally no reactive species are present and accordingly no chemical reaction should occur. Therefore, there would be no reason to protect reactive groups. Also in their own recent publication, the present inventors adhered to this scheme (Brandt et al. (2003)). In this publication, the inventors describe a method of on-chip purification of PNAs, wherein different reactivities of the polymer and the by-products are exploited, thereby allowing spotting onto a microarray and purification to occur concomitantly. However, and as noted above, this concomitant spotting and purification occurs after de-protection has taken place and implies irreversible covalent rebinding to a surface, thereby limiting the possible applications.

In the field of oligonucleotides, the trityl-on purification described further above departs from this scheme. It involves leaving the highly lipophilic triphenylmethyl (trityl) protection group attached to the desired oligonucleotide product, which is a hydrophilic molecule, thereby significantly modifying the separation properties of the desired product, and subsequently purifying the trityl-on oligonucleotide by HPLC in columns. More recently, gravity flow columns and small columns which can be arranged in microtiter plate format, both with reversed phase material for trityl-on oligonucleotide purification, have become available.

The present inventors were interested in a method of purifying PNAs and (poly)peptides, as well as PNA chimera and peptide-DNA chimera, and derivatives thereof. It is of note that PNAs are significantly more hydrophobic than oligonucleotides, as the negatively charged sugar-phosphate backbone of oligonucleotides is replaced by a peptide backbone bearing no charges in (poly)peptides and PNAs. Accordingly, attaching even a highly hydrophobic group such as the trityl group to a PNA is expected to result in a substantially smaller modification of the separation properties than its attachment to an oligonucleotide, and accordingly separation performance is expected to be unsatisfactory.

Surprisingly, the inventors were able to show that PNAs could be purified with a satisfactory discrimination power from synthesis by-products when the Fmoc protection group is left on ("Fmoc-on purification of PNA"). This result is surprising as PNAs are significantly more lipophilic than oligonucleotides. Secondly, the Fmoc group is less lipophilic than the trityl group. A sufficient modification of the purification properties of PNAs by leaving the Fmoc group on was therefore unexpected.

In a preferred embodiment of the methods of the invention, the logP value or the πₓ value, respectively, of said lipophilic group is greater than 1.0, preferably greater than 1.5, more preferred greater than 2.0, and most preferred greater than 3.0. However, also lipophilic groups with logP or πₓ values lower than 1.0, in particular with logP or πₓ values in the interval between zero and 1 are also within the scope of the present invention.

The lipophilic surface used in the method of the invention may be any surface that preferentially binds lipophilic molecules or molecules carrying lipophilic groups or substituents. With regard to shape, said surface may be planar or curved. Examples of curved surfaces are the surface of a sphere or microsphere. Other alternatives include wells of microtiter plates which are either coated and/or filled with lipophilic material. Said lipophilic material may also be a derivatised, lipophilic membrane.

The solution comprising the polymer to be purified may also comprise by-products of the polymer synthesis.

The skilled person knows how to choose the appropriate conditions for steps (a), (b) and (c), depending on the physico-chemical properties of the polymer, of the lipophilic group and of the lipophilic surface. For a polymer, wherein the polymer is a PNA, exemplary conditions are described in the examples enclosed herewith. Similar or identical conditions work also for (poly)peptides, PNA chimera and peptide-DNA chimera.

Obtaining the purified polymer from the washing solution from step (c) and collected in step (d) may be accomplished by methods well known in the art. Said methods comprise, for example, evaporation, lyophilization and/or precipitation.

The present invention also relates to a method of purifying a polymer selected from the group consisting of PNAs, (poly)peptides, PNA chimera, peptide-DNA chimera, and derivatives thereof, wherein the polymer carries at least one lipophilic group and wherein the method comprises the following steps: (a) transferring a solution comprising the polymer carrying a lipophilic group to a lipophilic surface under conditions that allow binding of said lipophilic group to said lipophilic surface; (b) washing said surface under conditions that allow said binding to be maintained, wherein polymers not carrying said lipophilic group are removed; (c) washing said surface under conditions that break said binding; (d) collecting the washing solution from step (c); and (e) obtaining said purified polymer from said washing solution.

In a preferred embodiment, said lipophilic group is a protection group.

In a more preferred embodiment, said protection group is a protection group used in the synthesis of the polymer. Accordingly, the polymer is obtained in protected form from the synthesis.

In a preferred embodiment, said lipophilic group is 9-Fluorenyl-methyl-oxy-carbonyl (9-Fluorenylmethoxy-carbonyl, Fmoc).

The presence of a lipophilic protection group on a polymer is inherent to the method of synthesizing the polymer and is exploited for the purpose of purification with superior discriminatory power according to the present invention. Alternatively, one may consider lipophilic groups or substituents attached during the course of synthesis, but not serving the purpose of protecting reactive functional groups. For example, many applications, in particular in the biotechnology field, require labelled polymers. A specific example are fluorescence-labelled PNAs for fluorescent *in situ* hybridization (FISH). Such labels may constitute groups or substituents with hydrophobic character whose potential for purification has not been recognized yet. Other applications require polymers with linkers, for example for the immobilization of the polymer on a carrier or support. Such applications include the manufacture of microarrays, wherein polymers such as DNA or PNA have to be immobilized on the surface of, for example, a coated glass slide. Such linkers, besides carrying a reactive functional group at their end needed for immobilization by either formation of a covalent bond or by non-covalent interactions, may exhibit a lipophilic portion, for example a straight or branched alkyl or an oligoaryl moiety.

Accordingly, in a preferred embodiment of the method of the invention the lipophilic group is a label and a purified labelled polymer is obtained in step (e).

The term "label" according to the present invention denotes a detectable substituent or detectable molecule. Detection methods comprise light absorption, fluorescence and luminescence. Molecules and substituents suitable as labels are well known in the art.

In a preferred embodiment, the method comprises the partial or complete removal of by-products of the synthesis of the polymer. Said by-products comprise polymers of shorter length than the desired polymer, i.e., polymers lacking one or more monomers.

The label, for example a fluorescent label, is bound to the polymer in the last cycle of the synthesis, for example by replacing a protection group, and serves subsequently, analogous to lipophilic protection groups such as Fmoc, as a lipophilic anchor (see Example 2).

In a more preferred embodiment, said label is a fluorescent dye.

In a more preferred embodiment, said fluorescent dye is selected from the group consisting of Cy5, Cy3, Alexa, Texas Red, fluoresceins and rhodamines.

In a further preferred embodiment, the lipophilic group is a linker and a purified polymer carrying at least one linker is obtained in step (e).

In a more preferred embodiment, the linker is selected from the group comprising carboxyalkanethioles, carboxyalkylamines and carboxyoligoarylthioles. The envisaged carboxyoligoarylthioles include linear chains of aryl rings with 2 to 10 members, wherein the aryl ring is phenyl, phenanthrenyl and/or anthracenyl.
Linkers carrying their respective standard protection groups and linkers without these protection groups are envisaged. Examples of protection groups for the thiol groups are the trityl and the MMT group.
Yet more preferred, the lipophilic group is an ω-carboxyalkanethiole or an ω-carboxyalkylamine.

In a further more preferred embodiment, the method further comprises the step of immobilizing the polymer on a support via the linker. For example, linkers carrying a thiol group may be immobilized on gold surfaces via the thiol group.

It is also envisaged to introduce a lipophilic group solely for the purpose of easier purification and/or purification with better discriminatory power. Accordingly, another preferred embodiment of the method of the invention uses a lipophilic group which is oligoaryl or alkyl. For example, the alkyl group may be a C8- to C18-alkyl chain with a cleavable linker. The term "oligoaryl" comprises linear chains of aryl rings with 2 to 10 members, wherein the aryl ring is phenyl, phenanthrenyl and/or anthracenyl. Also envisaged are anthracenyl derivatives and trityl derivatives as well as the 4,4'-dimethoxybenzhydryl group and the xanthyl group.

In a preferred embodiment of the method of the invention, said lipophilic surface consists of or comprises C18 material (octadecyl), C12 material (dodecyl), C8 material (octyl) or C4 material (butyl) or is an aryl- or polyaryl-modified surface. Further reversed phase materials known in the art are also envisaged as lipophilic surfaces according to the invention, for example, C1-, C2-, C3-, C5-, C6-, phenyl-, phenylether-, and phenylhexyl-derivatized surfaces. All groups may be covalently attached to a silicon atom of the chromatographic matrix.

In a further preferred embodiment, the method of the invention is effected in parallel. In other words, the purification of different polymers is effected simultaneously.

In a further preferred embodiment, purification is effected on a filter microtiter plate or on a microtiter plate with frits.
The inventors recognized the purification of PNAs, (poly)peptides, PNA chimera, peptide-DNA chimera, and derivatives thereof as a bottleneck in the overall workflow leading from building blocks, such as monomers or oligomers such as dimers and trimers, to the desired purified molecules, which cannot be obviated by the established HPLC procedures. By performing the purification in microtiter plates instead of by HPLC, an easy, fast and cost-effective method of producing a purified polymer is obtained, which is amenable to parallelization.
On the other hand, for applications where throughput is not critical, purification according to the invention may be effected in columns.

As opposed to labels and linkers, the usefulness of protection groups is restricted to synthesis and subsequent purification by the methods of the invention. Therefore, removal of the lipophilic protection group is a further envisaged step.

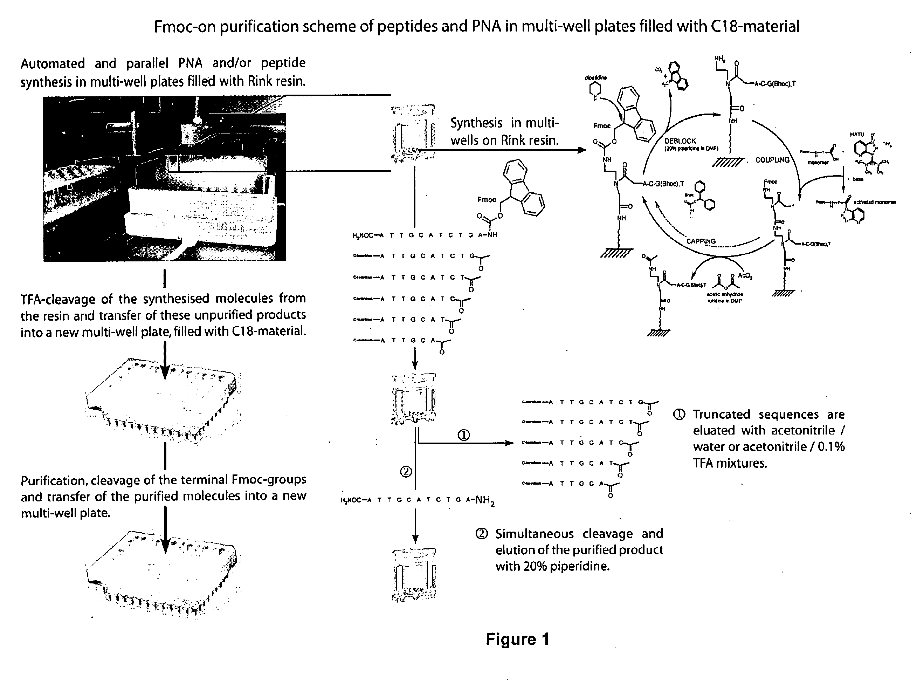

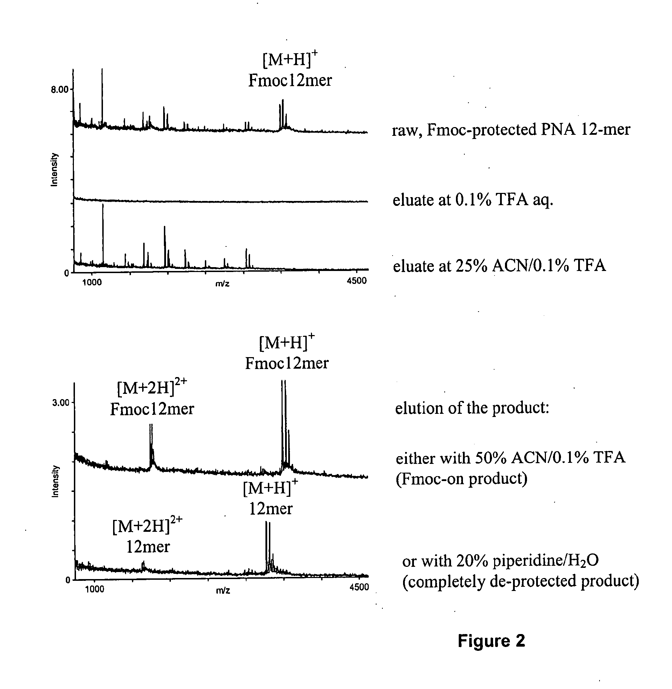

Accordingly, in a preferred embodiment of the method of the invention, step (c) is effected under conditions that cause cleavage of said protection group from the polymer, thereby obtaining purified de-protected polymer in step (e). Conditions suitable for (poly)peptides and PNAs are described in Example 1 and Figures 1 and 2.

Similarly, it is desirable to remove a lipophilic group introduced solely for the purpose of purification, once purification is accomplished.

Accordingly, in a preferred embodiment of the method of the invention, step (c) is effected under conditions that cause cleavage of said lipophilic group from the polymer, thereby obtaining purified polymer in step (e), wherein said purified polymer does not carry said lipophilic group.

The Figures show:
**Figure 1**: Fmoc-on purification scheme of peptides and PNAs in multi-well plates filled with C18 material
**Figure 2**: MALDI-TOF spectra exemplifying the course of the purification procedure

The following examples illustrate the invention but should not be construed as being limiting.

### Reference Example 1

### PNA synthesis

Synthesis of the PNAs was performed automatically by an AutoSpot robot (INTAVIS Bioanalytical Instruments AG, Cologne, Germany) in 96-well plates that have a frit in each well. A vacuum was applied to remove the reagents from the wells during the synthesis cycles. The Fmoc-protected ring resin LS (100-200 mesh, substitution of 0.2 mmol/g) was swelled for 1 h in N,N-dimethylformamide (DMF) (2 mg resin per 100 µl). The solution was thoroughly mixed and a volume of 100 µl was distributed to each well for a standard scale synthesis. After extraction of the DMF, Fmoc protection groups were removed from the resin by successive 1 min and 5 min incubations with 30 µl 20% (v/v) piperidine in DMF, with one DMF washing step in between. The resin was then washed five times with 80 µl DMF followed by the first coupling reaction. Per well, a volume of 4 µl Fmoc-protected PNA-monomer, biotin, AEEA-OH spacer or Cys(Mmt) (each 0.3 M in 1-methyl-2-pyrrolidone (NMP)), respectively, was activated for 60 sec with 2 µl HATU (0.54 M in DMF) and a 2 µl mix of N,N-diisopropylethylamine (DIEA, 0.6 M) and 2,6-lutidine (0.9 M) in DMF. Subsequently, the resin in each well was incubated with this mixture at room temperature for 20 min. Coupling was repeated after rinsing with DMF in between. The resin was then washed three times with DMF. For the capping of free, not elongated amino groups, there was an incubation with 5% acetic anhydride and 6% 2,6-lutidine in DMF for 5 min. Finally, the resin was washed another five times with 80 µl DMF. Deprotection, coupling of the next monomer and capping were repeated as described above until synthesis of the PNA-molecule was completed. For synthesis of fully acetylated PNAs, five additional acetylation steps were carried out after removal of the last Fmoc protection group.

Prior to cleaving the PNA-products from the resin, they were washed five times with 80 µl DMF followed by three washing steps with 80 µl 1,2-dichloroethane. After the resin was dried, 100 µl cleavage mix consisting of 80% trifluoroacetic acid (TFA) with 5% triisopropylsilane in 1,2-dichloroethane were added for the duration of 1 h at room temperature. Subsequently, the PNAs were processed in two different ways. They were either eluted from the resin with 200 µl water into another microwell plate, lyophilised and dissolved each in 100 µl water. Alternatively, the PNA was eluted from the resin with another 150 µl cleavage mix and subsequently precipitated twice with 1 ml ice cold diethyl ether. After remaining ether was evaporated, each PNA was dissolved in 100 µl water and stored at 4°C. For quality control, a 1 µl aliquot was diluted in 20 µl water and analysed by MALDI-TOF mass spectrometry. PNA-concentrations were calculated from OD values determined at 260 nm.

### Example 1

### Fmoc-on purification of PNA

In the following an exemplary workflow for the purification of PNAs is described with reference to Figure 1 enclosed herewith.
PNAs were synthesized in multititer plates with frits (alternatively on a resin or on a membrane as a carrier). The PNAs were cleaved from the carrier, wherein Fmoc remained bound ("Fmoc-on") and transferred to a further filter multititer plate using a vacuum station, wherein the wells of the filter multititer plate are filled with C18 material. Owing to the lipophilic properties of the Fmoc protection group, the full-length product exhibits a higher affinity to the C18 material as compared to the by-products of shorter length, which do not carry an Fmoc group, and elutes at a higher acetonitrile (ACN) contentration. The by-products are eluted at lower ACN concentration and discarded. Then the Fmoc-protected product may be eluted at a higher ACN concentration (not shown in Figure 1), or the lipophilic Fmoc group is directly cleaved off on the C18 material, wherein the complete de-protected product is eluted with 20% piperidine into a further multititer plate. The products purified thereby are lyophilized and, depending on the application, may be taken up in an appropriate solvent.
As the desired full-length product and the by-products belong to the same class of compounds and differ only slightly in their properties, it is generally difficult to separate the full-length product, in particular from the longer by-products, which are only slightly shorter than the full-length product. Leaving the Fmoc group on greatly increases the difference in the solubility and permits a more efficient separation.
The elution properties of Fmoc-protected PNAs as compared to the by-products have been determined by purifying aliquots of Fmoc-PNAs (4-, 12- and 16-mers, synthesized on a membrane (Jacob et al. (2003)) with C18-ZipTip pipet tips (Millipore). For this purpose only PNAs have been used, which, owing to bad synthesis conditions, were contaminated with a fraction of by-products, which is elevated as compared to normal synthesis conditions. The quality control of the synthesized raw products after cleavage from the membrane has been performed with MALDI-TOF MS. The flow-through (unbound material) and all eluates have also been analyzed with MALDI-TOF MS. For all PNAs assessed a satisfactory purification was achieved.
In the following the procedure for the PNA 12-mer AGCTTACGGATC is given:
1. ZipTip pipet tip with C18 material is washed and equilibrated with 3 x 5 µl 80% ACN/0.1 % TFA followed by 4 x 5 µl 0.1% TFA.
2. 5 µl of the raw product are taken up and the flow-through (unbound material) is kept and analyzed by MALDI-TOF MS.
3. By-products are eluted with 25% ACN/0.1 % TFA.
4. Elution with 50% ACN/0.1 % TFA yields the purified Fmoc-protected product. Alternatively, elution may be effected with 20% piperidine in water, which leads to cleavage of the Fmoc group and yields the complete de-protected product.
Figure 2 shows the MALDI-TOF spectra corresponding the steps described above.

A modified protocol was tested with PNA 13-mers, which were synthesized in multititer plates on a 0.4 µmol scale. In this case purification was done in 96-well plates with reversed-phase material from Merck (LiChroPLATE plates) or alternatively with POROS material (Applied Biosystems) or C18-coated porous SiO₂ beads (Grom).

### Example 2

### Purification of labeled PNA

PNAs or peptides carrying a terminal fluorescent dye can be purified using a similar protocol, wherein the dye replaces the Fmoc group as a lipophilic anchor. Depending on the dye, elution of the final product occurs at 50-80% ACN/0.1 % TFA.

### References

Becker et al. (1985). Use of a C4 column for reversed phase high performance liquid chromatographic purification of synthetic oligonucleotides. *J. Chrom.* **326**, 293-299.
Brandt et al. (2003). PNA microarrays for hybridisation of unlabelled DNA samples. *Nucleic Acids Res.* **31,** e119.
Casale et al. (1999). Synthesis of PNA oligomers by Fmoc chemistry. In Peptide Nucleic Acids: Protocols and Applications., Nielsen, P. E. (ed.). Horizon Bioscience, Wymondham, pp. 39-50.
Demidov et al. (1994). Stability of peptide nucleic acids in human serum and cellular extracts. *Biochem. Pharmacol.,* **48,** 1310-1313.
Demidov and Frank-Kamenitskii (2002). PNA openers and their applications. *Methods Mol. Biol.,* **208,** 119-130
Egholm et al. (1993). PNA hybridizes to complementary oligonucleotides obeying the Watson-Crick hydrogen-bonding rules. *Nature,* **365,** 566-568.
Husband et al. (2000). Development of simultaneous purification methodology for multiple synthetic peptides by reversed-phase sample displacement chromatography. *J. Chromatography A* **893,** 81-94.
Jacob et al. (2003). Production of PNA-arrays for nucleic acid detection. *Peptide Nucleic Acids; Protocols and Applications* (Nielsen, P.E., ed.), Horizon Bioscience, Wymondham, 261-279.
Jarowicki and Kocienski (2000). P., *J. Chem. Soc., Perkin Trans.* **1,** 2495-2527.
Koch (1999). PNA oligomers synthesis by Boc chemistry. In Peptide Nucleic Acids: Protocols and Applications., Nielsen, P. E. (ed.). Horizon Bioscience, Wymondham, pp. 21-38.
Kocienski (1994). Protecting Groups, Thieme Publishers, Inc.
Kuhn et al. (2002). Hybridisation of DNA and PNA molecular beacons to single-stranded and double-stranded DNA targets. *J. Am. Chem.* Soc., 124, 1097-1103.
Kuhn et al. (2003). Artificial site-specific DNA-nicking system based on common restriction enzymes assisted by PNA openers. *Biochemistry,* **42,** 4985-4992.
Larsen et al. (2003). Detection of gamma-globin mRNA in fetal nucleated red blood cells by PNA fluorescence *in situ* hybridisation. *Prenat. Diagn.,* **23,** 52-59.
Murdock et al. (2000). The age-related accumulation of a mitochondrial DNA control region mutation in muscle, but not brain, detected by a sensitive PNA-directed PCR clamping based method. *Nucleic Acids* Res., **28,** 4350-5.
Nielsen et al. (1991). Sequence-selective recognition of DNA by strand displacement with a thymine-substituted polyamide. *Science,* **254,** 1497-1500.
Orum et al. (1993). Single base pair mutation analysis by PNA directed PCR clamping. *Nucleic Acids Res.,* **21,** 5332-6.
Pipkorn et al. (2002). High-throughput peptide synthesis and peptide purification strategy at the low micromole-scale using the 96-well format. *J. Peptide Res.* **59,** 105-114.
Ray and Norden (2000). Peptide nucleid acid (PNA): its medical and biotechnical applications and promise for the future. *Faseb J.,* **14,** 1041-1060.
Rigby et al. (2002). Fluorescence *in situ* hybridization with peptide nucleic acid probes for rapid identification of Candida albicans directly from blood culture bottles. *J. Clin. Microbiol.,* **40,** 2182-2186.
Sangster (1997). Octanol-water Partition Coeffcients: fundamentals and physical chemistry, John Wiley & Sons, Chichester.
Sun et al. (2002). Detection of tumor mutations in the presence of excess amounts of normal DNA. *Nat. Biotechnol,* **20,** 186-9.
Wittung et al. (1994). DNA-like double helix formed by peptide nucleic acid. *Nature* **368**, 561-563.

## Claims

1. A method of producing a purified polymer selected from the group consisting of PNAs, (poly)peptides, PNA chimera, peptide-DNA chimera, and derivatives thereof, wherein the polymer carries at least one lipophilic group and wherein the method comprises the following steps:
(a) transferring a solution comprising the polymer carrying a lipophilic group to a lipophilic surface under conditions that allow binding of said lipophilic group to said lipophilic surface;
(b) washing said surface under conditions that allow said binding to be maintained, wherein polymers not carrying said lipophilic group are removed;
(c) washing said surface under conditions that break said binding;
(d) collecting the washing solution from step (c); and
(e) obtaining said purified polymer from said washing solution.

2. The method of claim 1, wherein said lipophilic group is a protection group.

3. The method of claim 1 or 2, wherein said lipophilic group is Fmoc.

4. The method of claim 1, wherein the lipophilic group is a label and wherein a purified labelled polymer is obtained in step (e).

5. The method of claim 4, wherein said label is a fluorescent dye.

6. The method of claim 5, wherein said fluorescent dye is selected from the group consisting of Cy5, Cy3, Alexa, Texas Red, fluoresceins and rhodamines.

7. The method of claim 1, wherein said lipophilic group is a linker and wherein a purified polymer carrying at least one linker is obtained in step (e).

8. The method of claim 7, wherein the linker is selected from the group comprising carboxyalkanethioles, carboxyalkylamines and carboxyoligoarylthioles.

9. The method of claim 7 or 8, further comprising the step of immobilizing the polymer on a support via the linker.

10. The method of any one of claims 1 to 9, wherein the lipophilic group is oligoaryl or alkyl.

11. The method of any one of claims 1 to 10, wherein said lipophilic surface consists of or comprises C18 material, C12 material, C8 material or C4 material or is an aryl- or polyaryl-modified surface.

12. The method of any one of claims 1 to 11, wherein the method is effected in parallel.

13. The method of any one of claims 1 to 12, wherein purification is effected on a filter microtiter plate or on a microtiter plate with frits.

14. The method of any one of claims 1 to 3 or 10 to 13, wherein step (c) is effected under conditions that cause cleavage of said lipophilic group from the polymer, thereby obtaining purified polymer in step (e), wherein said purified polymer does not carry said lipophilic group.
